# EUROPEAN PATENT APPLICATION

(11) **EP 3 412 677 A1**
(43) Date of publication of application: **12.12.2018**
(21) Application number: 17814304.6
(22) Date of filing: 07.04.2017
(51) Int. Cl.: C07F 9/44

(54) **CYCLOBUTYL (S)-2-[[[(R)-2-(6-AMINOPURIN-9-YL)-1-METHYL-ETHOXY]METHYL-PHENOXY-PHOSPHORYL]AMINO]-PROPANOATES, AND METHOD FOR PRODUCING AND USING SAME**

(30) Priority: 28.02.2017 RU 2017106615
(71) Applicant: Alla Chem, LLC, Hallandale Beach, FL 33009 (US); Ivachtchenko, Alexandre Vasilievich, Hallandale Beach, FL 33009 (US); Ivashchenko, Andrey Alexandrovich, Moscow 127576 (RU); Ivachtchenko, Alena Alexandrovna, Hallandale Beach, FL 33009 (US); Savchuk, Nikolay Filippovich, Rancho Santa Fe CA 92067 (US)
(72) Inventor: IVACHTCHENKO, Alexandr Vasilievich, Hallandale Beach, Florida 33009 (US); MITKIN, Oleg Dmitrievich, g. Khimki 141400 (RU)
(74) Representative: Zellentin & Partner mbB Patentanwälte
(86) International application number: PCT/RU2017/000212
(87) International publication number: WO 2018/160091

(57) **Abstract**

The present invention relates to chemotherapeutic agents for the treatment of viral and cancerous diseases. Said compounds are prodrugs of the inhibitors of human immunodeficiency virus (HIV) and hepatitis B virus (HBV) of DNA polymerase and are intended for the treatment of human immunodeficiency virus, hepatitis B and co-infections HIV/HCV, HIV/ HBV, HIV/HCV/HBV, and HCV/HBV.

## Description

### Field of the Invention

The present invention relates to chemotherapeutic agents for the treatment of viral and cancer diseases. These compounds are prodrugs of the inhibitors of human immunodeficiency virus (HIV), DNA polymerase hepatitis B virus (HBV), and DNA polymerase hepatitis C virus (HCV) and are intended to treat human immunodeficiency virus, hepatitis C, hepatitis B, and co-infections HIV/HCV, HIV/HBV, HIV/HCV/HBV, and HCV/HBV.

### Background of the Invention

The human immunodeficiency virus (HIV) belongs to the group of primate lentiviruses that are the etiologic agents of Acquired Immunodeficiency Syndrome (AIDS). The disease was first described in 1981, and HIV-1 was isolated by the end of 1983. Since then, AIDS has become a worldwide epidemic expanding in scope and magnitude as HIV infections have affected different groups of population and geographic regions. Around the globe, millions of people are now infected with HIV; once infected, individuals remain infected for life. Within a decade, the overwhelming majority of HIV-infected individuals left untreated develop fatal infections as a result of HIV-induced deficiencies in the immune system. AIDS is one of the world's most important public health problems at the start of the 21^{st} century. The development of highly active antiretroviral therapy (HAART) for chronic suppression of HIV replication and AIDS prevention has been a major achievement in HIV medicine [http://basicmedicalkey.com/aids-and-lentiviruses/].

HIV continues to be a major global public health issue. In 2015, 36.7 million people worldwide were living with HIV (of these, 1.8 million were children) - a global HIV prevalence of 0.8%. The vast majority of this number live in low- and middle-income countries. In the same year, 1.1 million people died of AIDS-related illnesses. According to experts' estimates, 78 million people have become infected with HIV and 35 million have died of AIDS-related illnesses since the epidemic began. An estimated 25.5 million HIV-infected people live in Sub-Saharan Africa. The overwhelming majority of them (estimated as 19 million) live in eastern and southern Africa, which saw 46% of new HIV infections globally in 2015. Around 40% of all people living with HIV do not know that they have the virus [http://www.avert.org/global-hiv-and-aids-statistics].

The development of antiviral drugs has significantly changed the perception of HIV/AIDS from a fatal to chronic and potentially manageable disease, and the availability and administration of antiretroviral therapy (ART) has significantly reduced mortality and morbidity associated with HIV and AIDS. There is a relationship between ART and the quality of life of people living with HIV and AIDS, and several studies have reported a strong positive association between ART and improved quality of life in different domains among people living with HIV and AIDS in both developed and developing countries [https://www.ncbi.nlm.nih.gov/pmc/articles/PMC3418767/].

HIV-infected patients are living longer lives in the era of highly active antiretroviral therapy (HAART). The performance of ART therapy may extend their lifespan by up to 70-80 years. However, concomitant infection with HBV and/or HCV leads to higher morbidity and mortality rates associated with liver diseases.

Uncontrolled HIV infection accelerates the progression of HCV-induced sclerosis of the liver.

HIV/HBV coinfection is a common phenomenon. Chronic HBV infection occurs in 5-10% of HIV-infected individuals exposed to HBV at a rate 10 times higher than the general population [http://hivinsite.ucsf.edu/InSite? page=kb-05-03-04#S1X].

HIV/HCV-coinfected patients have a three-fold greater risk of progression to cirrhosis or decompensated liver disease than HCV-monoinfected patients [https://aidsinfo.nih.gov/guidelines/html/1/adult-and-adolescent-arv-guidelines/26/hcv-hiv]. In a 2006 multinational cohort of more than 25000 HIV-infected persons in the United States and Europe, 14% of deaths were liver related and, of those, 66% occurred in persons with concomitant HCV infection [http://hivinsite.ucsf.edu/InSite? page=kb-00&doc=kb-05-03-05].

Monoinfection with either HBV or HCV represents one of the major causes of chronic liver diseases globally. However, in endemic areas a substantial number of patients are infected with both viruses mainly as a result of the common routes of transmission. Numerous studies have demonstrated that dually infected patients carry a greater risk of cirrhosis and hepatocellular carcinoma compared with monoinfected patients. Strikingly, approximately 60% of patients with inactive HBV infection before HCV treatment may present HBV reactivation while other HBV-infected patients experience hepatitis B surface antigen seroconversion [https://www.ncbi.nlm.nih.gov/pmc/articles/PMC4367211/].

Nucleosides (and nucleotides) have already been used in clinical practice for about 50 years and have become the cornerstone for the treatment of patients with viral infections or cancer. The development of some new medicinal preparations over the last decade has demonstrated that this class of compounds still shows considerable promise.

Nucleos(t)ides have played an important role in the treatment of viral diseases. They appear to be the basis of some multidrug regimens for HIV-infected patients. Today, nucleos(t)ides are the preferable option and the standard of treating HBV-infected patients.

A number of nucleos(t)ide RT inhibitors have been approved for the treatment of HIV-infection [R. F. Shinazi et al. Pharmacology of current and promising nucleosides for the treatment of human immunodeficiency viruses. J. Antiviral Res. 2006, 71, 322-334. E. De Clercq. The nucleoside reverse transcriptase inhibitors, nonnucleoside reverse transcriptase inhibitors, and protease inhibitors in the treatment of HIV infections (AIDS). Adva Pharmacol. 2013, 67, 317-358]. Some of them are applied in adjuvant therapy jointly with other HIV replication inhibitors providing convenient therapeutic regimens that have become the standard of care in highly active antiretroviral therapy (HAART). The drug combinations include Combivir®, Trizivir®, Epzicom®, Truvadas, Atriple, Stribile, and Compleras. Truvada, Atriple, Stribile, and Compleras comprise a nucleoside emtricitabine and an acyclic nucleotide tenofovir diisopropyl fumarate (TDF), while CombivirTM, Trizivir, and Epzicom include a combination of two or three drugs containing nucleosides Zidovudine (AZT), Lamivudine (3TC, Nuc21, and/or Abacavir (ABC) [R. F. Shinazi et al. Pharmacology of current and promising nucleosides for the treatment of human immunodeficiency viruses. J. Antiviral Res. 2006, 71, 322-334.]. The success of antiretroviral HIV therapy has dramatically increased the lifespan of individuals infected with this disease which used to be incurable. However, despite these advances, a quest is underway to discover new agents for treating chronically infected people and reducing resistance and side effects related to long-term drug administration.

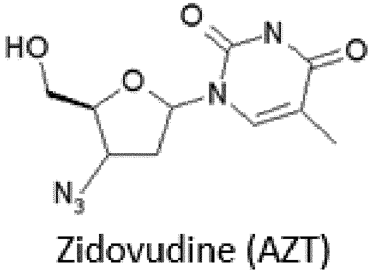

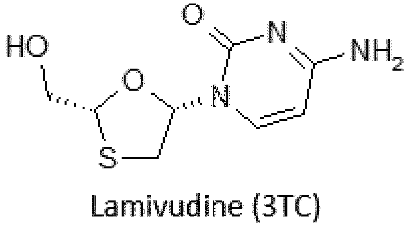

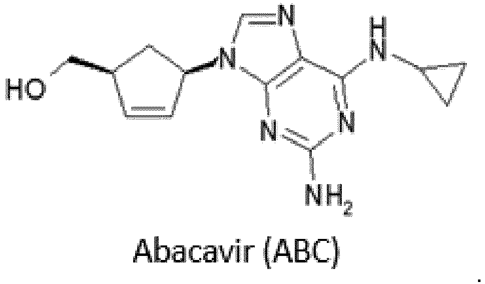

One of the novel antiviral acyclic nucleoside prodrugs is tenofovir phosphonate (TFV). Tenofovir disoproxil fumarate (TDF) as well as tenofovir alafenamide and its salts (TAF, GS-7340, Vemlidy) have shown even better properties. This prodrug is an antiviral medication designed precisely for the combination therapy treatment of patients in need thereof [WO 2002008241. US 7390791. A.S. Ray, M.W. Fordyce, M.J.M. Hitchcock. Tenofovir alafenamide: A novel prodrug of tenofovir for the treatment of Human Immunodeficiency Virus. Antiviral Research Volume 125, January 2016, Pages 63-70. WO 2002008241. US 7390791. WO2013025788, WO 2013116720, US 9296769. http://www.gilead.ca/pdf/ca/genvoya_pm_english.pdf. http://www.accessdata.fda.gov/drugsatfdadocs/nda/2015/207561 Orig1s000PharmR.pdf.]. 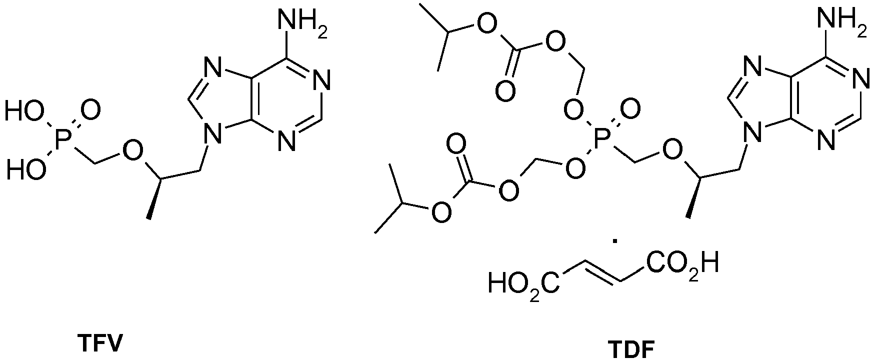 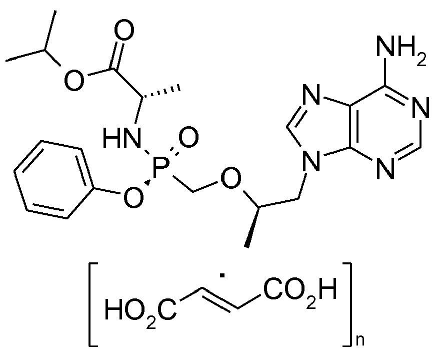
**n = 1: tenofovir alafenamide;**
**(TAF, GS-7340, Vemlidy);**
**n = 2: TAF fumarate;**
**n = 0.5: TAF hemifumarate.**

In 2015, FDA approved the first TAF-based composition for the combined treatment of HIV [http://www.dailykos.com/story/2013/4/10/1200735/-Gilead-s-New-FDA-Approved-HIV-Drug-Improves-Nothing-So-Naturally-It-Costs-A-Lot], and in 2016, TAF was approved by the FDA as a medicinal product for HBV treatment [https://www.hepmag.com/article/fda-approves-vemlidy-tenofovir-alafenamide-taf-hepatitis-b]. TAF is a powerful prodrug against the hepatitis B virus (HBV). As compared to TDF, TAF has less detrimental effects on the kidneys and bones [http://www.aidsmap.com/Tenofovir-alafenamide-works-well-against-hepatitis-B-with-less-effect-on-bones-and-kidneys/page/3051008/].

Despite the dramatic progress in the development of combined antiretroviral regimens to suppress long-lived HIV and HBV infections, novel safe medicinal products capable of sustaining high potency throughout patients' life are requisite.

### Summary of the invention

The inventors have surprisingly found that previously unknown cyclobutyl (*S*)-2-[[[(*R*)-2-(6-aminopurin-9-yl)-1-methyl-ethoxy]methyl-phenoxy-phosphoryl]amino]propanoate of general formula **1,** stereomer (cyclobutyl (*S*)-2-[(*S*)-[[(*R*)-2-(6-aminopurin-9-yl)-1-methyl-ethoxy]methyl-phenoxy-phosphoryl]amino]propanoate of formula **1.1** and cyclobutyl (*S*)-2-[(*R*)-[[(*R*)-2-(6-aminopurin-9-yl)-1-methyl-ethoxy]methyl-phenoxy-phosphoryl]amino]propanoate of formula **1.2,** isotopically enriched analogs, pharmaceutically acceptable salts, hydrates, solvates, and crystalline or polycrystalline forms thereof are more effective prodrugs of TFV that show promise for the combined treatment of viral diseases, especially, for HIV and viral hepatitises.

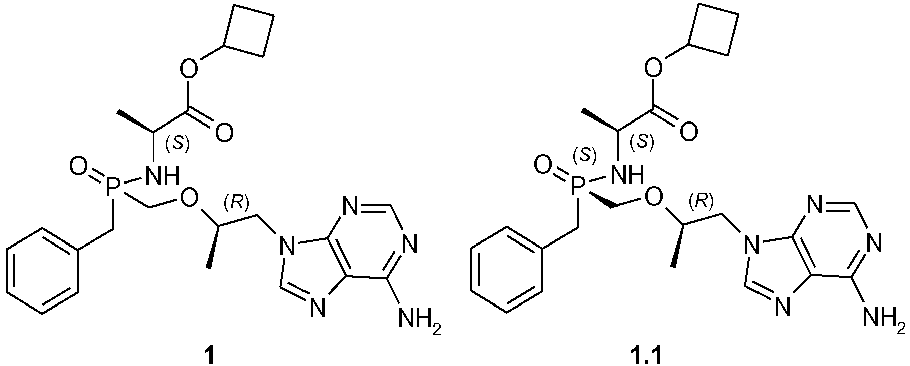

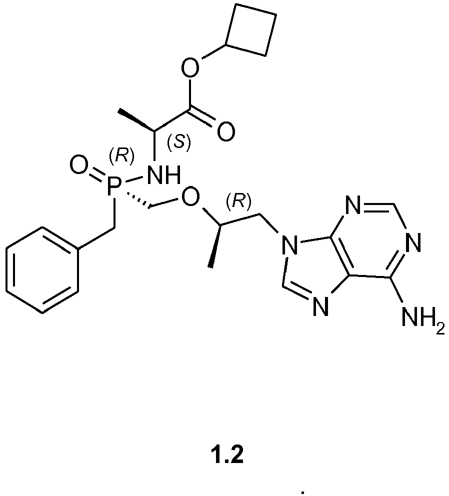

Listed below are definitions of various terms used to describe this invention. These definitions apply to the terms as they are used throughout this specification and claims, unless otherwise limited in specific instances, either individually or as part of a larger group.

The term "prodrug" refers to the compounds of this invention which have chemically or metabolically cleavable groups and become, by solvolysis or under physiological conditions, the compounds of this invention that are pharmaceutically active *in vivo.* Prodrugs often offer advantages of solubility, tissue compatibility, delivery, or delayed release in mammals (see, Bungard, H., Design of Prodrugs, pp. 7-9, 21-24, Elsevier, Amsterdam 1985). Prodrugs include acid derivatives well known to those skilled in the art, such as esters obtained by reaction of a starting acid compound with a suitable alcohol, or amides obtained by reaction of a starting acid compound with a suitable amine. Examples of prodrugs include, but are not limited to, acetate, formate, benzoate, and other acylated derivatives of alcohols or amines of functional groups in the compounds of this invention.

The term "active component" (drug substance) refers to a physiologically active compound of synthetic or other (biotechnological, plant , animal, bacterial, and so on) origins, which exhibits pharmacological activity and is an active ingredient of a pharmaceutical composition.

The term "crystalline form" refers to a substance structure wherein the molecules are arranged to form a crystal lattice.

The term "polycrystalline form" refers to a polycrystalline substance structure consisting of a plurality of monocrystals, or crystallites of certain crystalline form.

The term "medicinal drug " refers to a compound (or a mixture of compounds forming a pharmaceutical composition) in the form of tablets, capsules, injections, ointments, or other finished dosage forms intended for the restoration, improvement, or modification of physiological functions in humans and animals, and for the treatment and prophylaxis of diseases, for diagnostics, anesthesia, contraception, cosmetology, etc.

The term "therapeutic cocktail" refers to a simultaneously administered combination of two or more medicinal drugs that exhibit different mechanisms of pharmacological action and are directed at various biotargets taking part in the pathogenesis of disease.

The term "pharmaceutical composition" refers to a composition comprising a compound of general formula 2 and at least one of the components selected from the group consisting of pharmaceutically acceptable and pharmacologically compatible fillers, solvents, diluents, carriers, auxiliary, distributing, and receptive agents, excipients, delivery agents, such as preservatives, stabilizers, fillers, disintegrators, moisteners, emulsifiers, suspending agents, thickeners, sweeteners, flavoring agents, aromatizing agents, antibacterial agents, fungicides, lubricants, and prolonged delivery controllers, the choice and proportions of which depend on the nature and way of administration and dosage. Examples of suitable suspending agents are ethoxylated isostearyl alcohol, polyoxyethylene, sorbitol and sorbitol ether, microcrystalline cellulose, aluminum metahydroxide, bentonite, agar-agar and tragacant, and mixtures thereof.

Protection against microorganisms can be provided using various antibacterial and antifungal agents, such as parabens, chlorobutanol, sorbic acid, and the like. Said composition may also include isotonic agents, such as sugar, sodium chloride, and the like. The sustained action of the composition can be achieved using agents that decelerate the absorption of the active ingredient, for example, aluminum monostearate and gelatin. Examples of suitable carriers, fillers, solvents, diluents and delivery agents include water, ethanol, polyalcohols and mixtures thereof, natural oils (such as olive oil), and organic esters (such as ethyl oleate) for injections. Examples of fillers are lactose, milk sugar, sodium citrate, calcium carbonate, calcium phosphate, and the like. Examples of disintegrators and distributors are starch, alginic acid and salts thereof, and silicates. Examples of lubricants are magnesium stearate, sodium lauryl sulfate, talc, and polyethylene glycol of high molecular weight. A pharmaceutical composition for peroral, sublingual, transdermal, intramuscular, intravenous, subcutaneous, and local or rectal administration of the active ingredient, alone or in combination with another active compound, may be administered to animals and people in a standard administration form as a mixture with traditional pharmaceutical carriers. Suitable standard administration forms include peroral forms, such as tablets, gelatin capsules, pills, powders, granules, chewing gums, and peroral solutions or suspensions; sublingual and transbuccal administration forms; aerosols; implants; local, transdermal, subcutaneous, intramuscular, intravenous, intranasal, or intraocular forms; and rectal administration forms.

The term "inert filler" as used herein refers to a compound that is used for forming a pharmaceutical composition and is, as a rule, safe, nontoxic, and neither biologically nor otherwise undesirable, and comprises excipients acceptable for veterinary and human pharmaceutical use. Compounds of this invention may be administered individually but are generally administered in a mixture with one or more pharmaceutically acceptable excipients, diluents, or carriers chosen depending on the contemplated way of drug administration and standard pharmaceutical practice.

The term "pharmaceutically acceptable salt" refers to relatively nontoxic, both organic and inorganic salts of acids and bases claimed herein. Said salts can be obtained by *in situ* synthesis, isolation, or purification of compounds or they can be prepared specially. In particular, basic salts can be specially prepared from a purified free base of a compound claimed herein and a suitable organic or inorganic acid. Examples of salts thus prepared include hydrochlorides, hydrobromides, sulfates, bisulfates, phosphates, nitrates, acetates, dichloroacetates, oxalates, valeriates, oleates, palmitates, stearates, laurates, borates, benzoates, lactates, tosylates, citrates, maleates, fumarates, succinates, tartrates, mesylates, malonates, salicylates, propionates, ethanesulfonates, benzenesulfonates, sulfamates, and the like (a detailed description of the properties of said salts is given in Berge S.M., et al., "Pharmaceutical Salts" J. Pharm. Sci. 1977, 66: 1-19). The salts of the acids claimed herein may be also specially prepared by reaction of a purified acid with a suitable base to produce metal salts and amines. Said metal salts include the salts of sodium, potassium, calcium, barium, zinc, magnesium, lithium, and aluminum, of which sodium and potassium salts are preferable. Suitable inorganic bases used to produce metal salts include sodium hydroxide, carbonate, bicarbonate, and sodium hydride, potassium hydroxide and potassium bicarbonate, potassium carbonate; lithium hydroxide; calcium hydroxide; magnesium hydroxide; and zinc hydroxide. Suitable organic bases used to produce acid salts as claimed herein include amines and amino acids sufficiently basic to form a stable salt and suitable for medical use (in particular, they should be low-toxic). Said amines include ammonia, methylamine, dimethylamine, trimethylamine, ethylamine, diethylamine, triethylamine, benzylamine, dibenzylamine, dicyclohexylamine, piperazine, ethylpiperidine, tris(hydroxymethyl)aminomethane, and the like. Furthermore, salts can be prepared using tetraalkylammonium hydroxides, such as choline, tetramethylammonium, tetraethylammonium, and the like. Amino acids may be selected from basic amino acids: lysine, ornithine, and arginine.

The term "therapeutically effective amount," as used herein, refers to an amount of a substance, prodrug, or drug needed for alleviating the symptoms of the disease in the subject. The dose of a substance, prodrug, or drug will meet individual demands in each particular case. Said dose may vary in a wide range depending on numerous factors like the severity of the disease to be treated, the age and the general condition of the patient, other medicaments used for the patient's treatment, the mode and route of administration, and the experience of the attending doctor. For oral administration, the daily dose is approximately 0.01-10 g, including all the values in between, both in monotherapy and/or combination therapy. The preferred daily dose is around 0.1-7 g. As a rule, in order to alleviate or eliminate the virus, a higher loading dose is given at the beginning of treatment with a subsequent reduction of the dose to a level sufficient to prevent an infection burst.

The term "subject" refers to a mammal including, but not limited to, cattle, hogs, sheep, chickens, turkeys, buffalos, lamas, ostriches, dogs, cats, and humans; a human subject is most preferable. It is assumed that a subject's treatment may involve the use of any prodrug of general formula **1,** its stereomer, isotopically enriched analog, pharmaceutically acceptable salt, hydrate, solvate, and crystalline or polymorphic form or their combinations with another compound, including with an HCV NS5A inhibitor.

The term "solvate" refers to a complex or an aggregate formed by one or more molecules of a solute, i.e., a compound of this invention or a pharmaceutically acceptable salt thereof and one or more molecules of a solvent. Said solvates are typically crystalline solids having a fixed molar ratio of the solute and the solvent. Representative solvents include, but are not limited to, water, ethanol, isopropanol, acetic acid, and so on. When the solvent is water, the solvate formed is a hydrate.

The present invention relates to a novel prodrug of TFV-cyclobutyl (*S*)-2-[[[(*R*)-2-(6-aminopurin-9-yl)-1-methyl-ethoxy]methyl-phenoxy-phosphoryl]amino]propanoate of general formula **1,** its stereomers cyclobutyl (*S*)-2-[(*S*)-[[(*R*)-2-(6-aminopurin-9-yl)-1-methyl-ethoxy]methyl-phenoxy-phosphoryl]amino]propanoate of formula **1.1** and cyclobutyl (*S*)-2-[(*R*)-[[(*R*)-2-(6-aminopurin-9-yl)-1-methyl-ethoxy]methyl-phenoxy-phosphoryl]amino]propanoate of formula **1.2**), and their isotopically enriched analog, pharmaceutically acceptable salt, hydrate, solvate, and crystalline or polycrystalline forms.

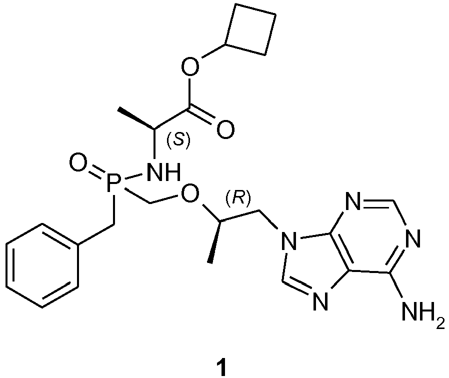

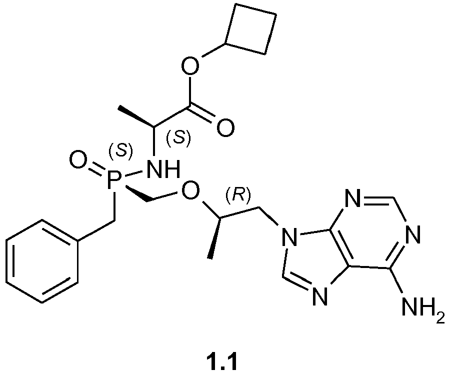

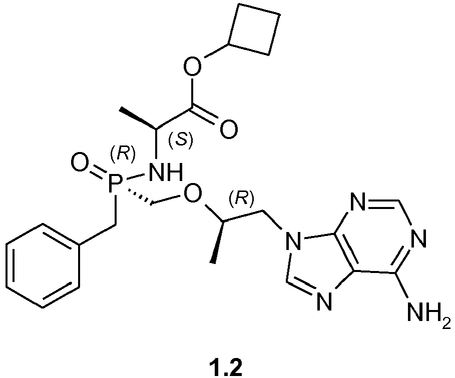

Preferable salts are fumarate, hemifumarate, dichloroacetate or hydrochloride of the compound of formula **1.**

More preferable salts are fumarate, hemifumarate, dichloroacetate or hydrochloride of the compound of formula **1.1.**

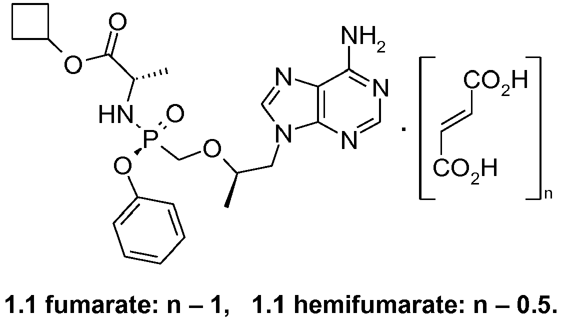

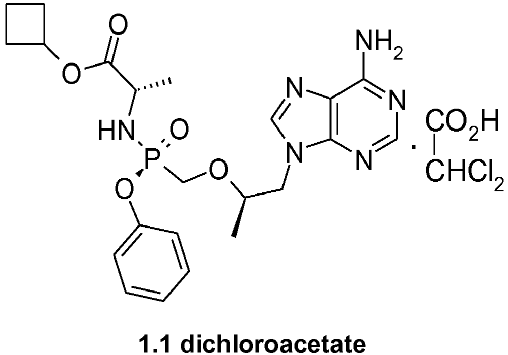

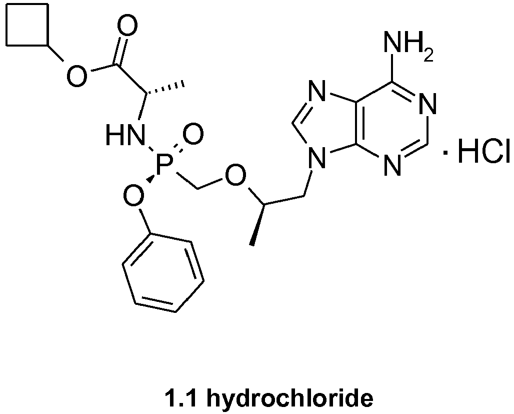

Surprisingly, the novel prodrugs of formula **1.1** and formula **1.2** appeared to be more effective than the known prodrug of TAF currently used in the combination therapy of HIV- and HBV-infected subjects. Indeed, the fumarates of the compounds of formulas **1.1** and **1.2** metabolize in the peripheral blood mononuclear cells (PBMCs) of TFV and TFV diphosphate leading to increased concentrations and AUCₗₐₛₜ of metabolites by contrast to those resulted from TAF metabolism in comparable conditions. As can be seen from Table 1, the TFV metabolite production rate of the prodrug of formula **1.1** Cₘₐₓ and AUCₗₐₛₜ of TFV diphosphate (drug) are almost twice as high as the corresponding values observed for TAF metabolism. Higher Cₘₐₓ and AUCₗₐₛₜ values (Table 1) are also observed for the metabolism of the prodrug of formula **1.2** as compared to corresponding values observed for TAF metabolism.

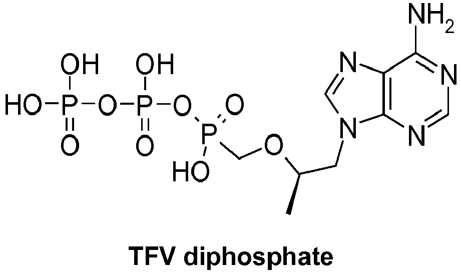

**Table 1. Pharmacokinetic metabolic parameters for the fumarates of formulas 1.1 and 1.2 and TAF in PBMC at initial prodrug concentrations of 30 µM**

| Prodrug | TFV metabolite formation | | | | TFV diphosphate metabolite formation | | | |
|---|---|---|---|---|---|---|---|---|
| | Cₘₐₓ, µM | AUCₗₐₛₜ, h·µM | Tₘₐₓ, h | T_{1/2}, h | Cₘₐₓ, µM | AUCₗₐₛₜ, h·µM | Tₘₐₓ, H | T_{1/2}, h |
| **1.1** | 0.28 | 13.1 | 4 | 30.7 | 1.76 | 82.6 | 24 | 21.4 |
| **1.2** | 0.13 | 7.27 | 24 | 64.5 | 1.24 | 63.4 | 24 | 65.1 |
| **TAF** | 0.11 | 5.86 | 4 | 65.9 | 0.96 | 49.4 | 24 | 73.2 |

The evaluation of antiviral activity for the fumarates of compounds **1.1** and **1.2** and TAF in a HIV test using SupT1 cells infected with the NL4.3 HIV strain containing a GFP-reporter (NL4.3GFP) virus has shown that the fumarate of the compound of formula **1.1** appears to be the most potent (Table 2) exhibiting both activity and selectivity 1.4 times higher than those of TAF.

**Table 2. Activity (EC₅₀), cytotoxicity (CC₅₀), and selectivity index (SI) for the fumarates of formulas 1.1 and 1.2 and TAF in an HIV test using SupT1 cells**

| Compound | EC₅₀, nM | CC₅₀, nM | SI = CC₅₀ / EC₅₀ |
|---|---|---|---|
| **1.1** fumarate | 27 | >100000 | >3704 |
| **1.2** fumarate | 38 | >100000 | >2632 |
| TAF | 35 | >100000 | 2857 |

The subject matter of the present invention is a pharmaceutical composition intended for the combination therapy and prophylaxis of viral infections and used in the form of tablets, capsules, or injections placed in a pharmaceutically acceptable package including compound of general formula **1,** or a stereomer thereof, or their isotopically enriched analog, pharmaceutically acceptable salt, hydrate, solvate, or crystalline or polymorphic forms.

Preferred is a pharmaceutical composition containing fumarate, hemifumarate, dichloroacetate or hydrochloride of the prodrug of formula **1.1** or an isotopically enriched analog, pharmaceutically acceptable salt, hydrate, solvate, or a crystalline or polycrystalline form thereof.

According to the present invention, said pharmaceutical composition may include a pharmaceutically acceptable filler and an additional therapeutic agent selected from the group consisting of a human immunodeficiency virus (HIV), inhibiting compounds protease , nonnucleoside reverse transcriptase HIV inhibitors, nucleoside reverse transcriptase HIV inhibitors, nucleotide reverse transcriptase HIV inhibitors, inhibitors of HIV-integrase, and CCR5 inhibitors.

A further subject matter of the invention is a method for the treatment of human immunodeficiency virus (HIV) including the administration to a subject in need thereof of a therapeutically effective amount of the compound of general formula **1** or a stereomer thereof, or an isotopically enriched analog, a pharmaceutically acceptable salt, a hydrate, a solvate, or a crystalline or polycrystalline form thereof.

A further preferred method for the treatment of human immunodeficiency virus (HIV) includes the administration to a subject in need thereof of a therapeutically effective amount of the compound of general formula **1.1** or an isotopically enriched analog, a pharmaceutically acceptable salt, a hydrate, a solvate, or a crystalline or polycrystalline form thereof.

A still further preferred method for the treatment of human immunodeficiency virus (HIV) includes the administration to a subject in need thereof of a therapeutically effective amount of fumarate, or hemifumarate, or dichloroacetate, or hydrochloride of the prodrug of formula **1.1** or an isotopically enriched analog, a hydrate, a solvate, or a crystalline or polycrystalline form thereof.

A still further preferred method for the treatment of human immunodeficiency virus (HIV) includes the administration to a subject in need thereof of a therapeutically effective amount of said pharmaceutical composition.

A further subject matter of the invention is a method for the treatment of human immunodeficiency virus (HIV) including the administration to a subject in need thereof of one or more additional therapeutic agents selected from the group consisting of inhibitors of the protease of human immunodeficiency virus (HIV), inhibiting compounds, nonnucleoside HIV inhibitors of reverse transcriptase, nucleoside HIV inhibitors of reverse transcriptase, nucleotide inhibitors of reverse transcriptase, inhibitors of HIV-integrase, and CCR5 inhibitors.

A still further subject matter of the invention is a method for the treatment of HBV infection of a therapeutically effective amount of the compound of general formula **1,** a stereomer thereof, or an isotopically enriched analog, a pharmaceutically acceptable salt, a hydrate, a solvate, or a crystalline or polycrystalline form thereof.

A preferred method for the treatment of HBV infection includes the administration to a subject in need thereof of a therapeutically effective amount of fumarate, or hemifumarate, or dichloroacetate, or hydrochloride of the prodrug of formula **1.1** or an isotopically enriched analog, a hydrate, a solvate, or a crystalline or polycrystalline form thereof.

A further subject matter of the invention is a method for the treatment of HBV infection including the administration to a subject in need thereof of a therapeutically effective amount of one or more additional therapeutic agents selected from the group consisting of the inhibitor of human immunodeficiency virus (HIV) protease, inhibiting compounds, nonnucleoside inhibitors of reverse HIV transcriptase, nucleoside inhibitors of reverse HIV transcriptase, nucleotide inhibitors of reverse transcriptase, HIV-interase inhibitors, and CCR5 inhibitors.

A further subject matter of the invention is a method for the treatment of human immunodeficiency virus (HIV) including the administration to the subject in need thereof of one or more doses of a therapeutically effective amount of the pharmaceutical composition mentioned above.

A further subject matter of the invention is a method for the production of cyclobutyl (*S*)-2-[[[(*R*)-2-(6-aminopurin-9-yl)-1-methyl-ethoxy]methyl-phenoxy-phosphoryl]amino]propanoate of general formula **1,** its stereomers (cyclobutyl (*S*)-2-[(*S*)-[[(*R*)-2-(6-aminopurin-9-yl)-1-methyl-ethoxy]methyl-phenoxy-phosphoryl]amino]propanoate of formula **1.1** and cyclobutyl (*S*)-2-[(*R*)-[[(*R*)-2-(6-aminopurin-9-yl)-1-methyl-ethoxy]methyl-phenoxy-phosphoryl]amino]-propanoate of formula **1.2,** and isotopically enriched analogs, pharmaceutically acceptable salts, hydrates, solvates, or crystalline or polycrystalline forms thereof including the use of L-alanine cyclobutyl ester of formula **2** and the compound of general formula **3**

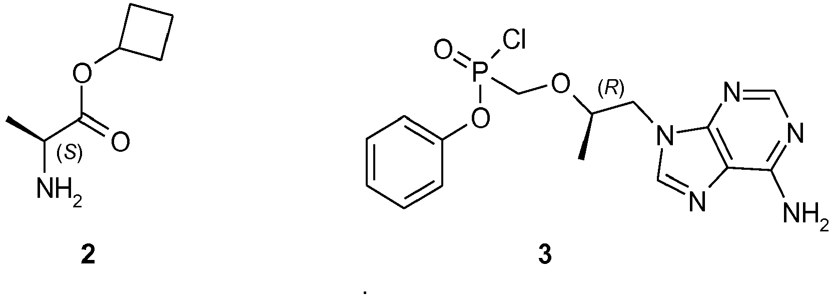

### Best embodiment

The present invention will now be described in terms of certain embodiments which are not intended to limit its scope. On the contrary, the present invention covers all alternatives, modifications, and equivalents that can be included within the scope of the claims. Thus, the following examples, which include specific embodiments, will illustrate this invention without limiting it.

**Example 1.** General synthetic protocol for the synthesis of the prodrugs of general formula **1** (Scheme 1).

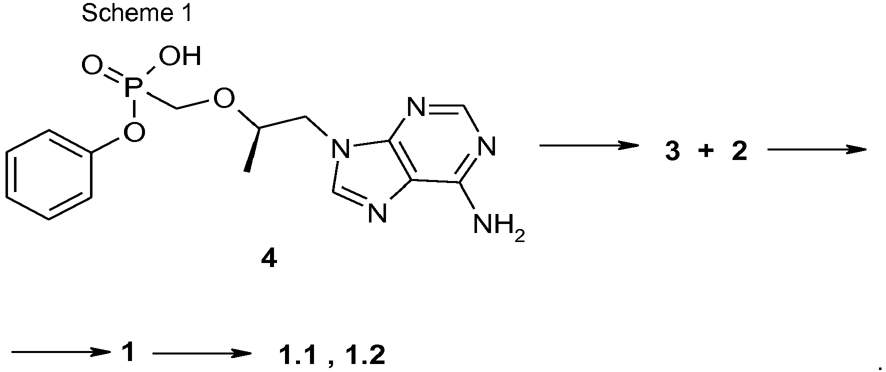

Thionyl chloride (3 ml, 40 mmol) was added dropwise with stirring to a suspension of [(R)-2-(6-amino-purin-9-yl)-1-methyl-ethoxymethyl]phosphonic acid monophenyl ether (3.63 g, 10 mmol) (**4**) [WO 2013116720] in sulfolane (14 ml) and dichloromethane (12 ml). The mixture was refluxed at 50-55°C under low Ar flow for 15 h. Then, vacuum (via a membrane pump) was delivered into the flask to remove volatile components for 2 h at 50-55°C. The reaction mixture was cooled down to 30°C, and a mixture of dichloromethane (10 ml) and dry acetonitrile (40 ml) was added with stirring. The reaction mixture containing chloride (**3**) was cooled down to (-60)-(-50)°C, and a solution of L-alanine cyclobutyl ester (**2**) (1.546 g, 12 mmol) and triethylamine (4.172 ml, 30 mmol) in 6 ml of acetonitrile was added. The mixture was slowly heated to room temperature, diluted with dichloromethane (100 ml) and the solution was spread onto about 100 ml of silica gel on a glass filter. The product was extracted by dry flash chromatography eluating first with dichloromethane, then with a 30% solution of acetone in dichloromethane, and finally with pure tetrahydrofurane to afford 2 g of the compound of general formula **1,** wherein the proportion of stereomers **1.1** and **1.2** was 2:3. The stereomers of formulas **1.1** and **1.2** were separated by HPLC on a Phenomenex Amylose-2 AXIA-Pac 250×21.20 MM optical column in an isocratic system of AcCN:EtOH:HCOOH 200:20:0.5 (flow rate 20 ml/min) with a 254-nm UV detector.

The fumarates of stereomers **1.1** and **1.2** were obtained by crystallization with an equimolar amount of fumaric acid from 100 ml of acetonitrile. The resulting products were fumarate (*S*)-cyclobutyl 2-((*S*)-(((*R*)-1-(6-amino-9*H*-purin-9-yl)propan-2-yloxy)methyl)(phenoxy)-phosphorylamino)-propanoate **(1.1),** LC-MS (ESI) 489 (M+H)⁺, ¹H NMR (DMSO-*d*₆, 300 MHz) δ 8.14 (s, 1H), 8.10 (s, 1H), 7.30 (m, 2H), 7.19 (s, 2H), 7.14 (m, 1H), 7.06 (m, 2H), 6.63 (s, 2H), 5.64 (t, *J* = 11.1 Hz, 1H), 4.86 (p, *J* = 7.2 Hz, 1H), 4.27 (dd, *J₁* = 14.4 Hz, *J₂* = 3.0 Hz, 1H), 4.14 (dd, *J₁* = 14.4 Hz, *J₂* = 6.6 Hz, 1H), 3.85 (m, 4H), 2.23 (m, 2H), 1.94 (m, 2H), 1.72 (m, 1H), 1.59 (m, 1H), 1.13 (d, *J* = 6.9 Hz, 3H), 1.07 (d, *J* = 6.6 Hz, 3H). ³¹P NMR (DMSO-*d*₆, 121.5 MHz) δ 22.05 and fumarate (*S*)-cyclobutyl 2-((*R*)-(((*R*)-1-(6-amino-9*H*-purin-9-yl)propan-2-yloxy)methyl)(phenoxy)phosphorylamino)propanoate **(1.2),** LC-MS (ESI) 489 (M+H)⁺, ¹H NMR (DMSO-*d*₆, 300 MHz) δ 8.14 (s, 1H), 8.12 (s, 1H), 7.34 (m, 2H), 7.21 (s, 2H), 7.15 (m, 1H), 7.11 (m, 2H), 6.63 (s, 2H), 5.53 (dd, *J₁* = 12.0 Hz, *J₂* = 10.5 Hz, 1H), 4.82 (p, *J* = 7.5 Hz, 1H), 4.29 (dd, *J₁* = 14.4 Hz, *J₂* = 3.6 Hz, 1H), 4.20 (dd, *J₁* = 14.4 Hz, *J₂* = 5.7 Hz, 1H), 3.98 (m, 1H), 3.86 (m, 3H), 2.21 (m, 2H), 1.91 (m, 2H), 1.69 (m, 1H), 1.57 (m, 1H), 1.13 (d, *J* = 6.9 Hz, 3H), 1.05 (d, *J* = 6.3 Hz, 3H). ³¹P NMR (DMSO-*d*₆, 121.5 MHz) δ 22.86.

**Example 2.** Preparation of a pharmaceutical composition in the form of tablet. Starch (1600 mg), ground lactose (1600 mg), talk (400 mg), and a salt of the prodrug of formula **1.1** (1000 mg) were mixed together and pressed into bar. The resulting bar was comminuted into granules and sifted through a sieve to collect granules of 14-16 mesh. The granules thus obtained were shaped into tablets of suitable form weighing 200 or 400 mg each.

**Example 3.** Preparation of a pharmaceutical composition in the form of capsules. The salt of the prodrug of formula **1.1** and lactose powder were carefully mixed in a ratio of 2:1. The resulting powdery mixture was packed into gelatin capsules of suitable size each weighing either 150 or 300 mg.

**Example 4.** Preparation of a pharmaceutical composition in the form of compositions for intramuscular, intraperitoneal, or hypodermic injections. The salt of the prodrug of formula **1.1** (500 mg), chlorobutanol (300 mg), propylene glycol (2 ml), and injectable water (100 ml) were mixed together. The resulting solution was filtered, placed into 5 ml ampoules, and sealed.

**Example 5.** Assessment of the metabolic parameters of prodrugs **1.1** and **1.2** and the **TAF** prototype in human peripheral blood mononuclear cells (PBMCs).

Generic solutions of tested compounds of formulas **1.1** and **1.2** and **TAF** were prepared in DMSO (Sigma) and kept at -20°C. The PBMCs (kept in liquid nitrogen before use) were extracted from human blood by means of Ficoll-Paque Premium (GE Healthcare) gradient centrifugation. The PBMCs were placed in 24-well plates (Greiner Bio-one), 1.5 mln cells per well (4.2 mln/ml), in the RPMI-1640 medium containing L-glutamine (2 mM), sodium private (0.11 mg/ml), essential and nonessential amino acids, penicillin 50 Un/ml, streptomycin (50 µg/ml) (all reagents by PanEco), and 5% HI (Heat Inactivated) fetal bovine serum (HyClone). The cells were incubated overnight at 37°C and 5% CO₂. The next day, tested and reference compounds in a final concentration of 30 µM were added to the cells. The cells and compounds were incubated at 37°C and 5% CO₂. After 2, 4, 8, 24, 48, and 72 hours of incubation, nonadherent cells were together with the medium transferred into 1.5 ml test tubes (Eppendorf) and centrifuged for 5 minutes at 1000g to remove the medium. The cells were washed with 1 ml of a phosphate buffer (Gibco) and lysed with 200 µl of 70% methanol cooled to -20°C. The cells that were adherent to the wells were washed with 1 ml of PBS (Gibco) and lysed with 200 µl of 70% methanol cooled to -20°C. The lysates of adherent and nonadherent cells from respective wells were combined and stirred.

The content of tenofovir (TFV) and diphosphate tenofovir (DP-TFV) in the cell lysates was determined by UPLC-MS/MS using a 1290 UPLC System (Agilent) chromatograph and a QTrap5500 System (AB Sciex) mass spectrometer with a triple quadrupole. The analytes were separated on a Thermo Hypercarb (50×3.0 mm, 5 µm, Thermo Scientific) column in a mobile phase comprising A - 0.5% ammonia in 25 mM of ammonium acetate and B - 0.5% ammonia in 25 mM of ammonium acetate: 2-propanol: methanol (1:1:3) at a flow rate of 0.8 ml/min. Electrospraying (TurbolonSpray) in a negative ion detection mode was used as an ion source. Analytes were detected in an MRM mode with transitions for TFV: 286>107, 286>79, 286>63 m/z and for DP-TFV: 446>348, 446>176, 446>158, 446>79 m/z. Chromatograms were analyzed using the Analyst 1.5.2 Software (AB Sciex). The concentrations of TFV and diphosphate TFV in cell lysates were estimated from calibration curves obtained using reference samples of TFV and DP-TFV in 70% methanol. The results are given in Table 1.

**Example 6.** Evaluation of anti-HIV activity of the prodrugs of general formula **1** and the prototype (TAF). The antiviral activity of tested compounds was evaluated on e T-lymphocytes line , SupT1. The cells were infected with HIV strain NL4.3 carrying a gene encoding the green fluorescent protein (NL4.3-GFP). A virus preparation was obtained by means of transfection of 293T cells of antiviral DNA. After 48 hours of transfection, the preparation was frozen and stored until being used. To increase the efficiency of infection, the suspension of SupT1 cells was sedimented from the infection mixture by centrifugation. Tested compounds were added to the cells immediately before adding the virus. After 2 hours of incubation, the infection mixture was replaced by a fresh culture medium with tested compounds. The efficiency of infection was evaluated after 45 hours by computing the percentage of fluorescence-bright cells against uninfected cell cultures. Concurrently, the cytotoxicity of tested compounds was evaluated in the same, but uninfected, cell line SupT1 using the XTT reagent. To determine antiviral activity and cytotoxicity, serial tenfold dilutions of the preparations were used (starting with 10 µM for antiviral activity and from 100 µM for cytotoxicity). DMSO (0.1%) was used for negative control. The values of EC₅₀, CC₅₀ and SI (selectivity index) were found. The quality of tests was evaluated based on the following controls: signal to background ratio, integrase inhibitor raltegravir (1 µM), and reproducibility of test results. Emetine (0.03, 0.09, and 0.2 µM) was used as the reference for cytotoxicity evaluation. The results are summarized in Table 2.

### Industrial applicability

The invention could be used in medicine and veterinary.

## Claims

1. Cyclobutyl (*S*)-2-[[[(*R*)-2-(6-aminopurin-9-yl)-1-methyl-ethoxy]methyl-phenoxy-phosphoryl]amino]propanoate of general formula **1,** cyclobutyl (*S*)-2-[(*S*)-[[(*R*)-2-(6-aminopurin-9-yl)-1-methyl-ethoxy]methyl-phenoxy-phosphoryl]amino]propanoate of formula **1.1,** and cyclobutyl (*S*)-2-[(*R*)-[[(*R*)-2-(6-aminopurin-9-yl)-1-methyl-ethoxy]methyl-phenoxy-phosphoryl]amino] propanoate of formula **1.2,** and isotopically enriched analogs, pharmaceutically acceptable salts, hydrates, solvates, crystalline or polycrystalline forms thereof

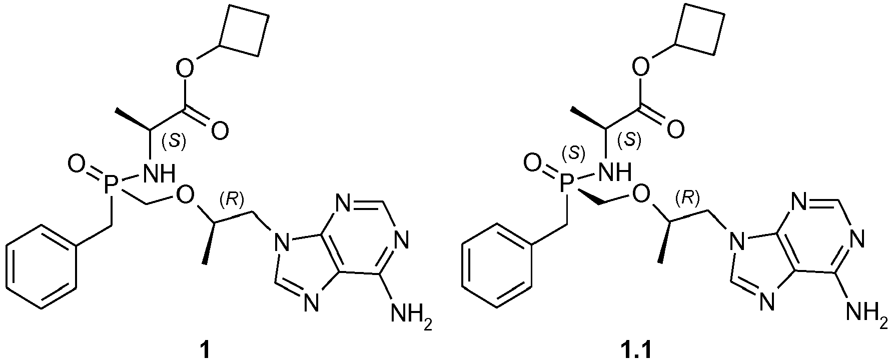

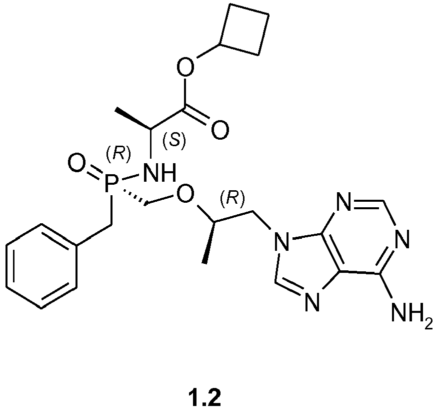

2. A compound according to Claim 1 is represented by fumarate, hemifumarate, dichloroacetate, or hydrochloride of formula **1.1**

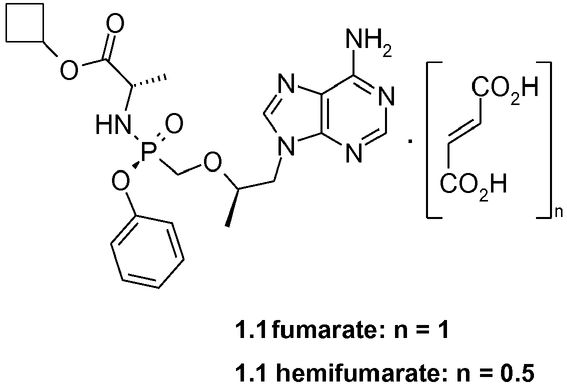

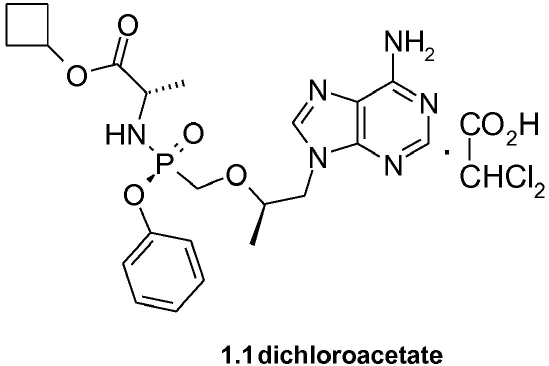

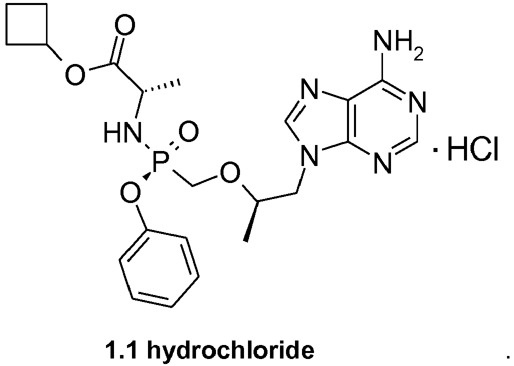

3. A pharmaceutical composition for the combination therapy and prophylaxis of viral infections in the form of tablet, capsules, or injections placed in pharmaceutically acceptable package comprising the compound of general formula **1,** or a stereomer thereof, or an isotopically enriched analog, a pharmaceutically acceptable salt, hydrate, solvate, or crystalline or polymorphic form thereof in a therapeutically effective amount.

4. The pharmaceutical composition according to Claim 3 containing fumarate, or hemifumarate, or dichloroacetate, or hydrochloride of the compound of formula **1.1** or an isotopically enriched analog, hydrate, solvate, or a crystalline or polymorphic form thereof.

5. The pharmaceutical composition according to Claims 3 or 4 additionally including one or more pharmaceutically acceptable fillers.

6. The pharmaceutical composition according to any of Claims 3, 4, or 5 additionally comprising one or more therapeutic agents selected from the group consisting of inhibitors of the protease of human immunodeficiency virus (HIV), nonnucleoside inhibitors of reverse HIV transcriptase, nucleoside inhibitors of reverse HIV transcriptase, nucleotide inhibitors of reverse HIV transcriptase, HIV-interase inhibitors, and CCR5 inhibitors.

7. A method for the combination therapy of human immunodeficiency virus (HIV) including the administration to the subject in need thereof of a therapeutically effective amount of the compound of general formula **1,** or a stereomer thereof, or an isotopically enriched analog, a pharmaceutically acceptable salt, hydrate, solvate, or crystalline or polymorphic form thereof.

8. The method for combination therapy according to Claim 7, wherein the stereomer is the compound of formula **1.1,** or a stereomer thereof, or an isotopically enriched analog, a pharmaceutically acceptable salt, hydrate, solvate, or a crystalline or polymorphic form thereof.

9. The method for combination therapy according to Claims 7 or 8, wherein the salt is fumarate, or hemifumarate, or dichloroacetate, or hydrochloride of the compound of formula **1.1** or an isotopically enriched analog, hydrate, solvate, or a crystalline or polymorphic form thereof.

10. The method for combination therapy of human immunodeficiency virus (HIV) including the administration of a therapeutically effective amount of the pharmaceutical composition according to Claims 3 - 6 to subject in need thereof.

11. The method for combination therapy according to any of Claims 7-9 including the administration to a subject of one or more additional therapeutic agents selected from the group consisting of the inhibitors of human immunodeficiency virus (HIV) protease, inhibiting compounds, nonnucleoside inhibitors of reverse HIV transcriptase, nucleoside inhibitors of reverse HIV transcriptase, nucleotide inhibitors of reverse transcriptase, HIV-interase inhibitors, and CCR5 inhibitors.

12. The method for combination therapy of hepatitis B virus (HBV) including the administration to a subject in need thereof of a therapeutically effective amount of the compound of general formula **1,** or a stereomer thereof, or their isotopically enriched analog, pharmaceutically acceptable salt, hydrate, solvate, or crystalline or polymorphic form.

13. The method for combination therapy according to Claim 12, wherein the stereomer is the compound of formula **1.1,** or an isotopically enriched analog, a pharmaceutically acceptable salt, a hydrate, a solvate, or a crystalline or polycrystalline form thereof.

14. The method for combination therapy according to Claims 12 or 13 wherein the salt is fumarate, or hemifumarate, or dichloroacetate, or hydrochloride of the compound of formula **1.1** or their isotopically enriched analog, hydrate, solvate, or crystalline or polycrystalline form.

15. The method for the combination therapy of hepatitis B virus (HBV) including the administration to a subject in need thereof of a therapeutically effective amount of the pharmaceutical composition according to Claims 3-6.

16. The method for combination therapy according to any of Claims 12-15 including the administration to a subject in need thereof of one or more additional therapeutic agents selected from the group consisting of the human immunodeficiency virus (HIV) protease inhibitors, inhibiting compounds, nonnucleoside inhibitors of reverse HIV transcriptase, nucleoside inhibitors of reverse HIV transcriptase, nucleotide inhibitors of reverse transcriptase, HIV-interase inhibitors and CCR5 inhibitors.

17. The method for the combination therapy of human immunodeficiency virus (HIV) according to any of Claims 7-11 including the administration to the subject in need thereof of one or more doses of the compound according to Claims 1 and 2 or the pharmaceutical composition according to Claims 3-6.

18. The method for the combination therapy of hepatitis B virus (HBV) according to any of Claims 12-16 including the administration to the subject in need thereof of one or more doses of the compound according to Claims 1 and 2 or the pharmaceutical composition according to Claims 3-6.

19. A process for the preparation of cyclobutyl (*S*)-2-[[[(*R*)-2-(6-aminopurin-9-yl)-1-methyl-ethoxy]methyl-phenoxy-phosphoryl]amino]propanoate of general formula **1,** cyclobutyl (*S*)-2-[(*S*)-[[(*R*)-2-(6-aminopurin-9-yl)-1-methyl-ethoxy]methyl-phenoxy-phosphoryl]amino]propanoate of formula **1.1,** cyclobutyl (*S*)-2-[(*R*)-[[(*R*)-2-(6-aminopurin-9-yl)-1-methyl-ethoxy]methyl-phenoxy-phosphoryl]amino]-propanoate of formula **1.2,** as well as isotopically enriched analogs, pharmaceutically acceptable salts, hydrates, solvates, or crystalline or polycrystalline forms thereof, including the use of L-alanine cyclobutyl ester of formula **2** and the compound of general formula **3**
